# EUROPEAN PATENT APPLICATION

(11) **EP 2 778 176 A1**
(43) Date of publication of application: **17.09.2014**
(21) Application number: 13305280.3
(22) Date of filing: 12.03.2013
(51) Int. Cl.: C07K 16/44, C07K 7/06, C07K 17/06

(54) **Compositions and methods for analyzing histidine phosphorylation**

(71) Applicant: SANOFI, 75008 Paris (FR); Salk Institute For Biological Studies, La Jolla, CA 92037 (US)
(72) Inventor: The designation of the inventor has not yet been filed
(74) Representative: Gaslonde, Aude

(57) **Abstract**

A peptide of the general structure: 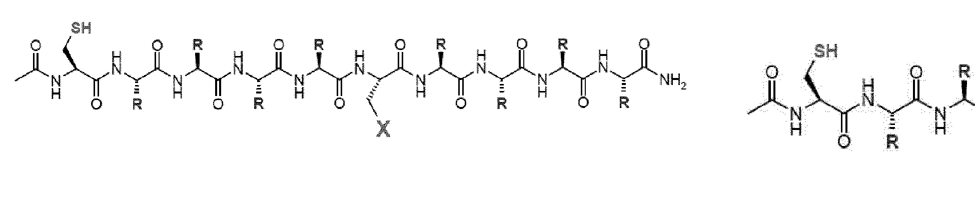 is provided, wherein X is a non--hydrolyzable pHis analogue and R is H or CH₃. Such peptides can be used to produce sequence-independent anti-phosphohistidine antibodies. Also provided is a composition comprising antibodies that specifically bind to said peptide when X is a phosphohistidine (or a non--hydrolyzable pHis analogue) but fail to specifically bind to said peptide when X is histidine.

## Description

### BACKGROUND

Protein phosphorylation regulates virtually all cellular processes and aberrant phosphorylation is the underlying cause of numerous cancers. The majority of intracellular proteins are phosphorylated at any given time, and, while 9 of the 20 amino acids can be phosphorylated, Ser, Thr, and Tyr phosphorylation has attracted the majority of attention in eukaryotic organisms. Histidine (His) phosphorylation has long been implicated in signal transduction (e.g. prokaryotic "two-component" systems); however, its role in mammalian cells remains largely unexplored. This is due to the difficulty in studying His phosphorylation by standard biochemical techniques.

Unlike pTyr, pSer and pThr, phosphohistidine (pHis) is heat and acid labile. Consequently, the importance of His phosphorylation has been greatly underestimated. Despite the brief half-life of pHis under acidic conditions (18-25 sec half-life in 1 M HCl, 49°C), the high-energy, phosphoramidate bond of pHis is stabilized by basic conditions, and the half-life in protein substrates is strongly influenced by neighboring amino acid residues indicating it is highly context dependent (12 day half-life of histone H4 at RT, pH 7.6). Thus, new tools are needed to bring this under-appreciated post-translational modification to light.

Despite the paucity of knowledge about His phosphorylation in eukaryotic signal transduction, there is growing evidence implicating His kinases in cancer and tumor metastasis. In fact, the first metastasis suppressor gene identified (by its reduced expression in highly metastatic melanoma cell lines) is one of only two known mammalian His kinases; Nm23-H1 (AKA NME1 or nucleoside diphosphate kinase [NDPK-A]). Nm23 family members are involved in intracellular nucleotide homeostasis as well as in both physiological and pathophysiological cellular processes such as proliferation, differentiation, development, apoptosis, cytokinesis and metastasis, through mechanisms that remain largely unknown.

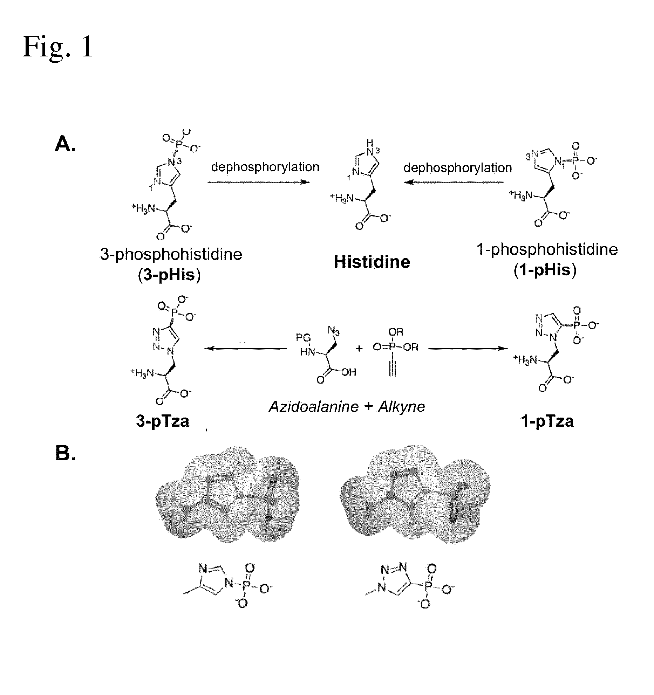

Nm23-H1 and the closely related Nm23-H2 (NME2/NDPK-B) catalyze transfer of phosphate from ATP onto NDPs through a pHis enzyme intermediate. Nm23-H1/- H2 also possess His kinase activity, transferring the phosphate from the active site pHis onto a His in a target protein. However, the lack of pHis-specific antibodies (Abs) and pHis's instability under typical conditions used for proteomics have made it difficult to study the role His phosphorylation plays in suppression of metastasis. Phosphospecific Abs exist for pSer, pThr and pTyr, and these, combined with biochemical and proteomic techniques, have proved invaluable in the study of protein phosphorylation in cellular signaling and cancer. Until recently, the difficulties in creating stable pHis peptides have precluded generation of pHis specific Abs. Development of non-cleavable pHis analogues now makes this possible [Kee et al., (2010) J Am Chem Soc. 132, 14327-9 and McAllister et al., (2011) Chem Commun. 47, 1297-9]. These pHis analogues will be used as immunogens to make Abs specific for both biologically relevant pHis isomers, 1- and 3-pHis (Fig. 1). Anti-1- and 3-pHis Abs will serve as novel tools to study His kinase activity of Nm23 proteins as well as yet undiscovered His kinases and their substrates.

It has been estimated that 6% of phosphorylation in eukaryotes occurs on His and that pHis could be 10-100 times more abundant than pTyr. Despite this, only a handful of mammalian pHis proteins, two His kinases (Nm23-H1/-H2) and a single pHis phosphatase (PHPT1) have been identified. For the few known pHis substrates, this phosphorylation has proved essential to their function and revealed novel signaling pathways. Nm23-H2 phosphorylates KCa3.1 (H358) and is required for potassium channel activation. Phosphorylation of heterotrimeric Gs protein subunit β1 (H266) by Nm23-H2 activates Gs and regulates basal cAMP accumulation. Furthermore, Nm23-H2, G proteins and caveolin expression are mutually dependent for stable localization and caveolae formation. Histone H4 phosphorylation (H18) is associated with enhanced cell proliferation in liver and thymus. The development of pHis-specific Abs combined with improved techniques for pHis peptide enrichment and identification by MS will be used to greatly expand the number of known pHis targets and determine which ones play a role in suppression of tumor metastasis by His kinases. In addition, this protocol may identify novel His kinases, since known His kinases autophosphorylate on His.

### SUMMARY

As disclosed herein peptides are provided that comprise non-hydrolyzable phosphohistidine analogues, 1-pTza & 3-pTza, for use as immunogens (see Fig. 1). Such peptides, comprised only of immunogenically neutral amino acids with small side chains (e.g., alanine and glycine) and a non-hydrolyzable phosphohistidine analogue, are used in generating antibodies that bind to proteins that comprise a phosphohistidine, but fail to bind to proteins of identical sequence wherein the histidine residue is not phosphorylated. In one aspect of the present disclosure, peptides comprising a library of random sequences (Lam, K. et. al., (1991) Nature, 354, 82- 4) of immunogenically neutral amino acids with short side chains (e.g., alanine and glycine) in addition to one of the two non-hydrolyzable pHis analogues are used to generate sequence-independent anti-pHis Abs.

The present disclosure is also directed to methods of generating sequence-independent anti-pHis antibodies as well as the sequence-independent anti-pHis antibodies themselves.

### BRIEF DESCRIPTION OF THE DRAWINGS

Fig. 1. Non-hydrolyzable pHis analogues: 3-pTza and 1-pTza
   Structures of phosphohistidine isomers; 3- and 1-phosphohistidine (3-pHis and 1-pHis). Non-hydrolyzable 3-pHis and 1-pHis analogues, 1- phosphoryltriazolylalanine (1-pTza) and 3-phosphoryltriazolylalanine (3-pTza), are synthesized via a "click chemistry" reaction between an azidoalanine and an alkyne. The same building blocks with different catalysts are used to make both isomers.

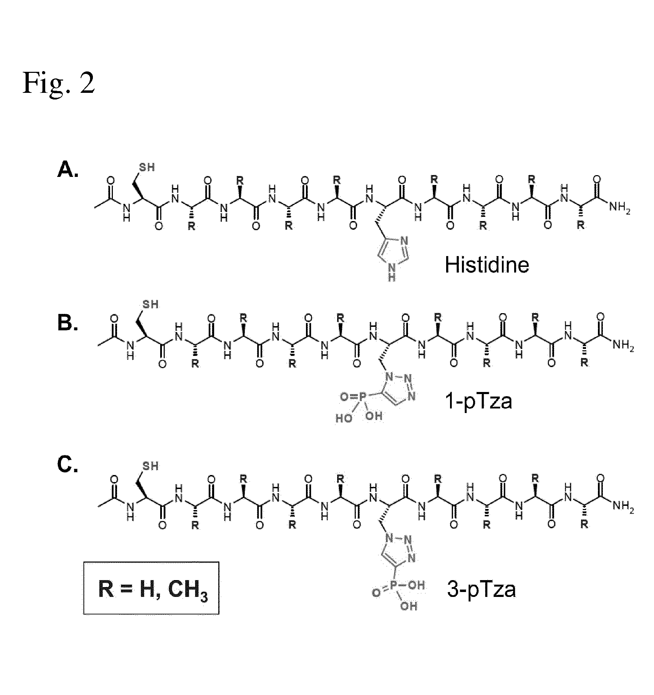

Fig. 2 Incorporation of the pHis analogues 1-pTza and 3-pTza into immunizing peptides. Three synthetic peptide libraries have been synthesized, consisting of either histidine or a non-hydrolyzable pHis analogue flanked by randomized, non-immunogenic amino acids (glycine [R=H] and alanine [R=CH3]), to promote generation of sequence-independent anti-pHis Abs. Each library is a complex mixture of peptides (2⁸ = 256) that are acylated at the N-terminus and contain an N-terminal L-cysteine for coupling to a carrier protein such as keyhole limpet hemocyanin (KLH). A. The library incorporating histidine will be used for negative selection of Abs that recognize non-phosphorylated histidine. B. & C. The immunizing peptide libraries, one for each isomer, contain the non-hydrolyzable analogues, 1-pTza or 3-pTza.

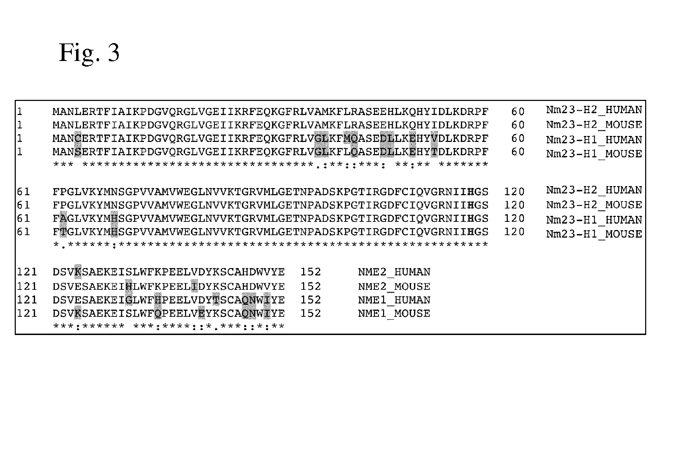

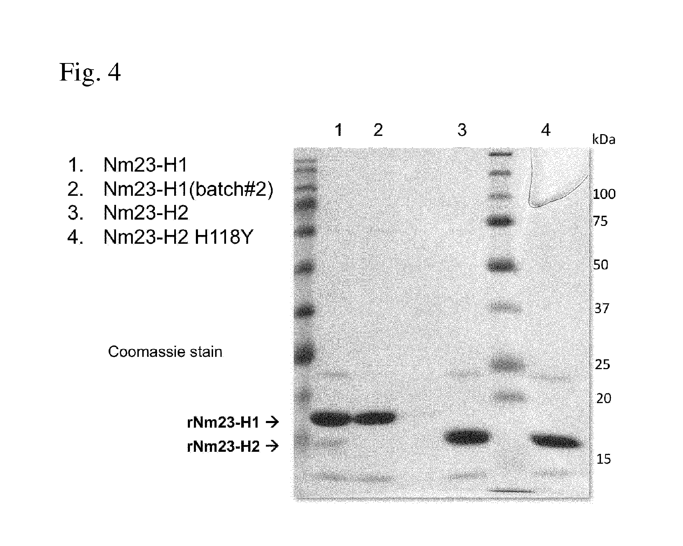

Fig. 3 Amino acid sequence alignment of the histidine kinases Nm23-H1 and Nm23-H2. Human Nm23-H1 and Nm23-H2 proteins are 88% identical. Human and mouse Nm23-H1 are 98% identical and human and mouse Nm23-H2 are 94% identical. Non-conserved residues are highlighted in grey. The catalytic, autophosphorylation site at H118 is highlighted in blue. Nm23-H1 and -H2 autophosphorylate themselves on the catalytic His residue (H118). The kinases themselves can therefore serve as positive and negative controls for testing the specificity of the rabbit antisera in applications such as immunoblotting and immunoprecipitation. The recombinant proteins Nm23-H1 and -H2, as well as their H118Y mutants, have been purified and analyzed for contaminating proteins by Coomassie staining (Fig. 4).
Fig. 4 Purification of recombinant Nm23-H1 & -H2
   Wild type (WT) and the autophosphorylation site, catalytic mutant (H118Y) of Nm23-H1 and Nm23-H2 were cloned into a GST fusion vector (pGEX-6P-1), transformed into the BL21 derivate, Rosetta 2(DE3) and expressed by IPTG induction. GST-tagged Nm23 proteins were purified with glutathione resin (GenScript). Nm23 proteins were released from the resin by cleavage of GST tags. Purification and complete cleavage of GST tag was assessed by SDS-PAGE and Coomassie staining. As expected, recombinant Nm23-H1 and Nm23-H2 migrate at 18 kDa and 16 kDa respectively.

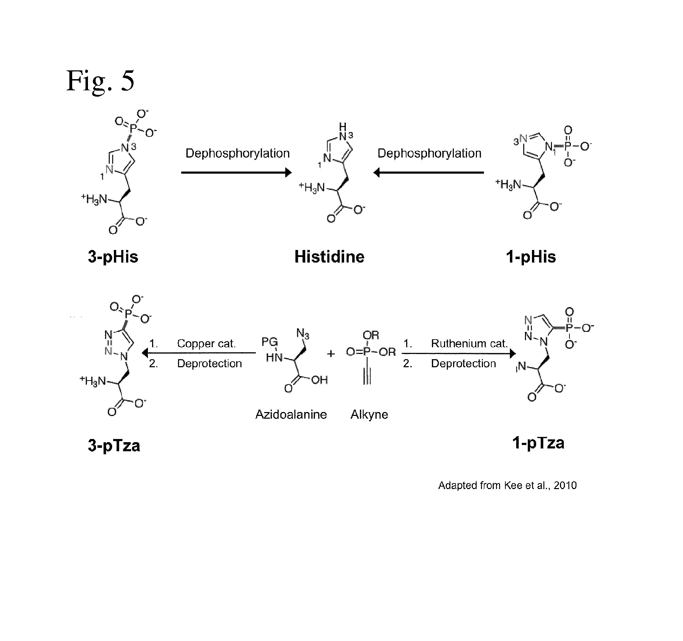

Fig. 5 Structures of 1- and 3-pHis and design of non-hydrolyzable pHis analogs. Fig. 5 provides the general synthetic scheme used to prepare the non-hydrolyzable phosphohistidine amino acid analogs.

### DETAILED DESCRIPTION

### DEFINITIONS

In describing and claiming the invention, the following terminology will be used in accordance with the definitions set forth below.

The term "about" as used herein means greater or lesser than the value or range of values stated by 10 percent, but is not intended to limit any value or range of values to only this broader definition. Each value or range of values preceded by the term "about" is also intended to encompass the embodiment of the stated absolute value or range of values.

As used herein, the term "pharmaceutically acceptable carrier" includes any of the standard pharmaceutical carriers, such as a phosphate buffered saline solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

As used herein, the term "treating" includes prophylaxis of the specific disorder or condition, or alleviation of the symptoms associated with a specific disorder or condition and/or preventing or eliminating said symptoms.

The term "antibody" or "antibodies" as used herein refers to all types of immunoglobulins, including IgG, IgM, IgA, IgD, and IgE, including Fab or antigen-recognition fragments thereof. The antibodies may be monoclonal or polyclonal and may be of any species of origin, including (for example) mouse, rat, rabbit, goat, sheep, pig, chicken, horse, or human, or may be chimeric antibodies, or camelid (single chain) antibodies. See, e.g., M. Walker et al., Molec. Immunol. 26: 403-11 (1989); Morrison et al., Proc. Nat'1. Acad. Sci. 81: 6851 (1984); Neuberger et al., Nature 312: 604 (1984)). The antibodies may be recombinant monoclonal antibodies produced according to the methods disclosed in U.S. Pat. No. 4,474,893 (Reading) or U.S. Pat. No. 4,816,567 (Cabilly et al.), or by recombining variable regions into expression vectors. The antibodies may also be chemically constructed according to the method disclosed in U.S. Pat. No. 4,676,980 (Segel et al.).

The term "specifically bind" means a special and precise interaction between two molecules which is dependent upon their structure, resulting in a high preference of binding between the two molecules relative to other molecules.

As used herein the term "patient" without further designation is intended to encompass any warm blooded vertebrate domesticated animal (including for example, but not limited to livestock, horses, cats, dogs and other pets) and humans.

### EMBODIMENTS

### DETAILED DESCRIPTION

As disclosed herein a library of random peptide sequences is provided wherein each peptide sequence comprises an analog of phosphohistidine wherein the phosphate group is stably linked as a phosphonate to a carbon in the histidine analog. More particularly, two non-hydrolyzable phosphohistidine analogues, 1-pTza & 3-pTza, respectively (see Fig. 1), have been incorporated into synthetic peptides for use in generating antibodies specific for proteins that comprise a phosphohistidine. In one aspect of the present disclosure peptides comprised of only immunogenically neutral amino acids (e.g., alanine and glycine) and a non-hydrolyzable pHis analogue are used to promote generation of sequence-independent anti-pHis Abs. In one embodiment the non-hydrolyzable pHis analogue is 1-pTza or 3-pTza. In a further embodiment the peptide comprises an amino acid that is linked to a hapten carrier protein. In one embodiment the carrier protein is keyhole limpet hemocyanin. In a further embodiment the 1-pTza or 3-pTza containing peptide further comprises a lysine, tyrosine or cysteine amino acid having a carrier protein linked to this amino acid. In one embodiment the 1-pTza or 3-pTza containing peptide comprises a cysteine covalently linked to keyhole limpet hemocyanin through a disulfide bond formed with the cysteine side chain.

In accordance with one embodiment a peptide is provided with the general formula Z-W-Y, wherein Z and Y are amino acid sequences comprised of immunogenically neutral amino acids having short side chains, and independently ranging in length from about 2 to about 8 amino acids in length, and W is a non-hydrolyzable pHis analogue. In one embodiment the amino acids are either glycine or alanine; however, any amino acid (natural or synthetic) can be used that tends not to induce a specific immune response. In one embodiment the non-hydrolyzable pHis analogue is 1-pTza or 3-pTza. In one embodiment the peptide comprises an amino acid that can be used for covalent attachment of additional compounds including, for example, a carrier protein. In one embodiment an additional amino acid is added at the amino or carboxy terminus of the peptide of the formula Z-W-Y wherein the additional amino acid is cysteine, tyrosine or lysine. In one embodiment the peptide is further modified to include an N-terminal acylation, and/or C-terminal amidation.

In one embodiment the peptide comprises the structure

Cys-Z-W-Y

wherein
Z is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈;
W is an amino acid selected from 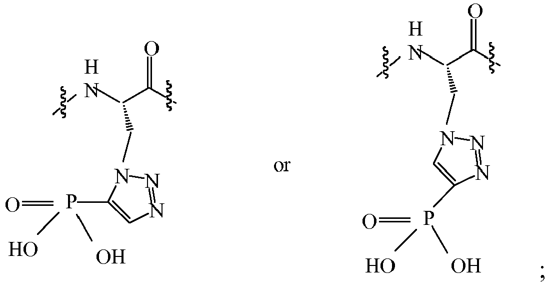 Y is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈; and
X₁, X₂, X₃, X₄, X₅, X₆, X₇, and X₈ are independently alanine or glycine. In one embodiment the N-terminal amine is acylated and the C-terminal carboxylic acid group is replaced with an amide. In another embodiment Z and Y are each independently selected from the group consisting of X₁X₂X₃X₄, X₁X₂X₃X₄X₅, X₁X₂X₃X₄X₅X₆, and X₁X₂X₃X₄X₅X₆X₇. In one embodiment both Y and W are independently X₁X₂X₃X₄. The peptide in some embodiment further comprises a carrier protein linked to the peptide, and in one embodiment the carrier protein is keyhole limpet hemocyanin covalently linked to the side chain of the cysteine of said peptide. In accordance with the present invention any carrier protein known to those skilled in the art can be used in accordance with the embodiment disclosed here.

In one embodiment a composition is provided comprising a plurality of peptides that differ in their primary sequence but conform to the general structure of Z-W-Y as defined herein. Such a composition is called a library. In one embodiment a library including at least 256 different peptides wherein each of said 256 peptides has the structure Cys-Z-W-Y or Z-W-Y is provided, wherein
Z is a sequence selected from the group consisting of X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈;
W is an amino acid selected from 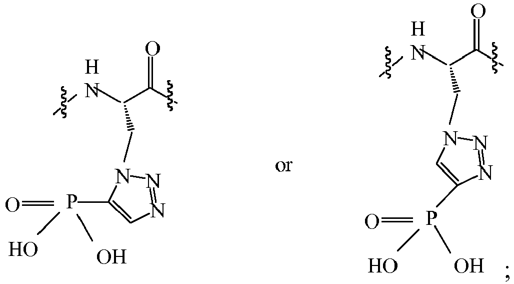 Y is a sequence selected from the group consisting of X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈; and
X₁, X₂, X₃, X₄, X₅, X₆, X₇, and X₈ are independently alanine or glycine. In a further embodiment the N-terminal amine is acylated and the C-terminal carboxylic acid group is replaced with an amide.

In one embodiment two separate libraries of peptides are provided. The first representing a 1-Phosphohistidine Mimetic Peptide Library and having the general structure:

AcCys-A/G-A/G-A/G-A/G-**1-pTza**-A/G-A/G-A/G-A/G-CONH₂

and the second library representing a 3-Phosphohistidine Mimetic Peptide Library and having the general structure:

AcCys-A/G-A/G-A/G-A/G-**3-pTza**-A/G-A/G-A/G-A/G-CONH₂,

wherein Ac = acylated N-Terminus, Cys = L-Cysteine for coupling antigens to KLH, A = Alanine, G = Glycine. The designation A/G indicates that either amino acid can be used at that position, thus each library contains a mixture of 2⁸ = 256 peptides. In one embodiment the peptides are synthesized under conditions where an alanine or glycine is randomly added to the growing peptide chain during synthesis. 1-pTza and 3-pTza = stable phosphohistidine analogues (Kee et al., J. Am. Chem. Soc., 2010)

In accordance with one embodiment the general structure of the 1-pTza and 3-pTza Peptide Libraries is as follows, wherein X is a non--hydrolyzable pHis analogue 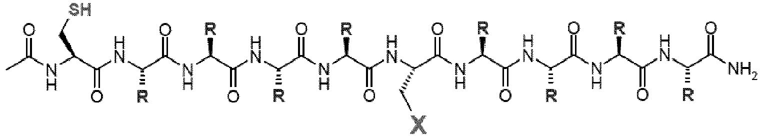 wherein R is H or CH₃.

More particularly, in one embodiment the structure of the 1-pTza Peptide Library is 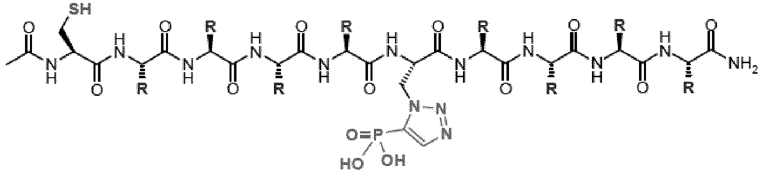 and the structure of the 3-pTza Peptide Library is

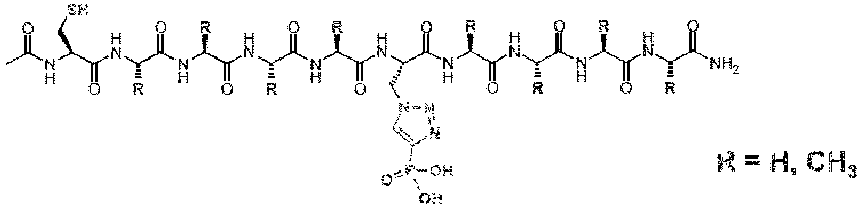

The peptides disclosed herein can be used to generate sequence-independent anti-pHis antibodies. In accordance with one embodiment a method for producing sequence-independent anti-pHis antibodies is provided wherein the method comprises
i) immunizing a host with a peptide of the general structure Z-W-Y as disclosed herein; and
ii) obtaining an antibody-containing host serum produced as a response to said immunization.

In one embodiment the host is immunized with a composition comprising a peptide of the general structure Z-W-Y wherein
Z is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈;
W is an amino acid selected from 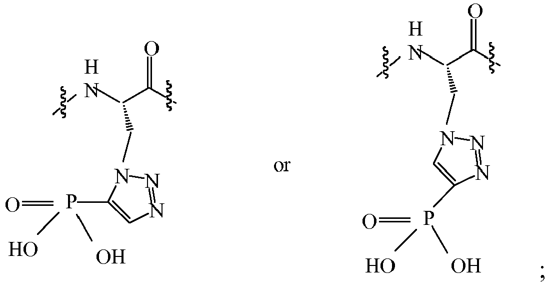 Y is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈; and
X₁, X₂, X₃, X₄, X₅, X₆, X₇, and X₈ are independently alanine or (glycine . In a further embodiment each of the peptides of the libraries are covalently linked to a carrier protein such as KLH. In one embodiment each of the peptides of the libraries comprises one or more peptide selected from the group consisting of lysine, cysteine and tyrosine wherein the carrier protein is linked to the side chain of the lysine, cysteine and/or tyrosine amino acid. Composition comprising the libraries(with or without the covalently linked carrier protein) can be optionally combined with an adjuvant to enhance the immunogenic response when the composition is injected into an animal. In one embodiment the adjuvant is Freund's Complete or Incomplete Adjuvant that is mixed/emulsified with the peptide-carrier protein prior to being injected.)

In one embodiment the host is immunized with a 1-pTza Peptide Library of compounds having the general structure of 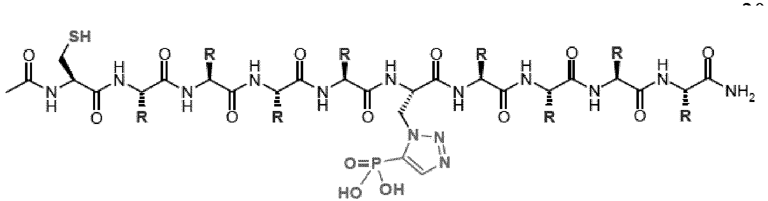 or with a 3-pTza Peptide Library of compounds having the general structure of

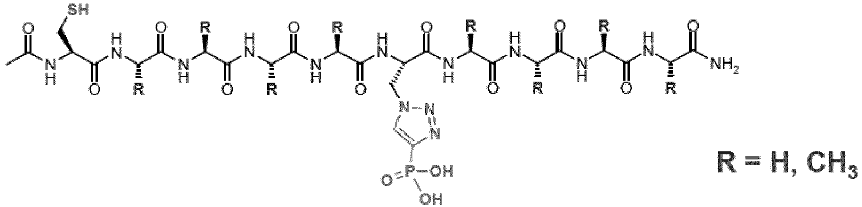

In one embodiment the peptides comprising the pTza Peptide Library further include a covalently bound carrier protein such as KLH, to create a hapten antigen. Any adjuvant known to those skilled in the art can be used in the method of generating antibodies. In one embodiment the adjuvant is selected from the group consisting of Bacille Calmette-Guérin, Complete Freund's adjuvant, Incomplete Freund's adjuvant and QS-21 (QS-21 is a purified plant extract that enhances the ability of the immune system to respond to vaccine antigens. It is derived from the Soap bark tree.).

Polyclonal antibodies of the invention may be produced according to standard techniques by immunizing a suitable animal (e.g., rabbit, goat, etc.) with an antigen encompassing a peptide for the general formula Z-W-Y as disclosed herein, covalently linked to a carrier protein, and emulsified with an adjuvant. Immune serum is collected from the animal after immunization using standard techniques, and the polyclonal antibodies are then isolated from the immune serum, in accordance with known procedures of affinity purification. The present invention also encompasses the antibodies produced by applicants' invention.

Mouse monoclonal antibodies of the invention may be produced in a hybridoma cell line according to the well-known technique of Kohler and Milstein. Nature 265: 495-97 (1975); Kohler and Milstein, Eur. J. Immunol. 6: 511 (1976); see also, CURRENT PROTOCOLS IN MOLECULAR BIOLOGY, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse and, after a sufficient time (in keeping with conventional techniques), the animal is sacrificed and spleen cells obtained. The spleen cells are then immortalized by fusing them with myeloma cells, typically in the presence of polyethylene glycol, to produce hybridoma cells. Alternatively, rabbit fusion hybridomas may be produced as described in U.S. Pat. No. 5,675,063, C. Knight, Issued Oct. 7, 1997. The rabbit spleen cells are fused with a (previously patented) suitable fusion partner to create an immortal cell line which is then grown in a suitable selection media, such as one including hypoxanthine-aminopterin-thymidine (HAT), and the cell supernatant screened for monoclonal antibodies having the desired specificity. The secreted antibody may be recovered from tissue culture supernatant by conventional methods such as precipitation, ion exchange or affinity chromatography, or the like.

Monoclonal Fab fragments may also be produced in *Escherichia coli* by recombinant techniques known to those skilled in the art. See, e.g., W. Huse, Science 246: 1275-81 (1989); Mullinax et al., Proc. Nat'l Acad. Sci. 87: 8095 (1990). If monoclonal antibodies of one isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Natl. Acad. Sci., 82: 8653 (1985); Spira et al., J. Immunol. Methods, 74: 307 (1984)). The invention also provides hybridoma clones that produce monoclonal antibodies specific for any protein containing a phosphohistidine. The heavy and light chain sequences from a mouse or rabbit hybridoma can be cloned and sequenced, and introduced into both eukaryotic and prokaryotic expression vectors to produce recombinant antibodies.

In accordance with the present disclosure the antibodies produced by immunizations with the peptides disclosed herein will specifically bind to peptides comprising a phosphohistidine, but will not specifically bind to an identical peptide that comprises a histidine in place of the phosphohistidine. In one embodiment the antibody is polyclonal and in another embodiment the antibody is monoclonal. The present invention also encompasses any hybridoma cell line producing such antibody. Accordingly, the antibodies of the present disclosure will specifically bind to histidine bearing amino acid sequences only when the protein is phosphorylated at the histidine (i.e, the protein comprises a 1- or -3-pHis). In one embodiment the antibody will specifically bind to an amino acid sequence of at least 3, 4, 5, 8 or 10 amino acids in length that bears a phosphohistidine, optionally an internal phosphohistidine. In one embodiment the antibody will specifically bind to an amino acid sequence bearing a phosphohistidine, optionally an internal phosphohistidine, wherein the amino acid sequence has a maximum length of 1,000, 800, 600, 500, 400, 300, 200, 100, 50, 25, 15, 10 or 5 amino acids.

In accordance with another embodiment the disclosure also provides a method for screening biological samples to detect proteins comprising a phosphohistidine. The method comprises the steps of:
(a) incubating/mixing a biological sample with at least one antibody that specifically binds to peptides comprising a phosphohistidine (i.e., will not bind to an identical peptide that comprises a histidine in place of the phosphohistidine) under conditions suitable for formation of a reagent-antibody complex (phosphohistidine-antibody complex) ; and
(b) detecting the presence of said complex in said sample, wherein the presence of said complex indicates the presence of a protein comprising a phosphohistidine in said sample. In one embodiment the antibody is labeled. Means for producing a detectable signal include the use of radioactive labels, fluorescent labels, enzyme labels, and so forth. Examples of suitable immunoassays are the radioimmunoassay, immunofluorescence methods, enzyme-linked immunoassays, imuno-(western) blotting, immunoprecipitation, and the like.

The peptides and antibodies disclosed herein may be conjugated to a solid support suitable for a diagnostic assay (e.g., beads, plates, slides or wells formed from materials such as latex or polystyrene) in accordance with known techniques. Antibodies of the invention, may likewise be conjugated to detectable groups such as radiolabels (e.g., ³H, ¹⁴C, ³³P, ³⁵S, ¹²⁵I, ¹³¹I), enzyme labels (e.g., horseradish peroxidase, alkaline phosphatase), far-red laser-activated dyes, and fluorescent labels (e.g., fluorescein) in accordance with known techniques.

In one embodiment a kit is provided for detecting proteins that comprise a phosphohistidine. In one embodiment the kit comprises a polyclonal or monoclonal antibody that specifically binds to peptides comprising a phosphohistidine. In one embodiment the kit further comprises a secondary antibody conjugated to a detectable label. The kit may alternatively or in addition include one or more containers, e.g., vials, tubes, bottles, and the like, optionally containing the antibody and assay reagents in a lyophilized form or in an aqueous solution. Preferably, the kits will also include instructions for use.

### EXAMPLE 1.

### Generation of Sequence Independent pHis Antibodies

Histidine exists as two tautomers and undergoes rapid tautomerization, with a hydrogen present at either N1 or N3 of the imidazole ring. Consequently, phosphorylation can occur at either N1 (1-pHis) or N3 (3-pHis) of the imidazole ring and isomerization can occur between N1 and N3. The local hydrogen-bonding environment of target His residues likely determines specificity of pHis at N1 or N3 in proteins.

Unsuccessful attempts have been made to generate pHis antibodies - pHis containing peptide immunogens are hydrolyzed too rapidly to elicit an immune response. In 2010, two groups (Muir - Rockefeller & Webb - Leeds) successfully synthesized stable analogs of pHis (pTza). The Muir group successfully incorporated the analogs into synthetic peptides, and raised the first sequence-specific pHis antibody (histone H4). pHis substrates lack an obvious pattern/motif in flanking amino acids and so it has been difficult to generate pHis antibodies that recognize more than one single kinase target sequence.

To generate sequence independent pHis antibodies, non-hydrolyzable phosphohistidine analogs were synthesized and then incorporated into synthetic peptides containing these pHis analogs flanked by randomized Gly and Ala.

These peptide libraries were coupled to KLH, emulsified with Freund's adjuvant, and injected into New Zealand White rabbits. The rabbits will be bled and antibodies recovered by affinity purification. Validation of the recovered anti-1-pHis and anti-3-pHis polyclonal antibodies was conducted based on standard immunoblot and immunoprecipitation procedures using purified pHis proteins and cell lysates.

### Materials and Methods

Reagents and their sources were as follows: KLH for peptide conjugation (Calbiochem; catalog no. 374817), Rosetta 2 (DE3) Competent Cells (Novagen; Catalog no. 71397), Glutathione Resin (GenScript; catalog no. L00206), PreScission Protease (GE Healthcare; catalog no. 27-0843-01), PBS blocking buffer with 1% Casein (BioRad; catalog no. 161-0783). Freund's Complete Adjuvant and Freund's Incomplete Adjuvant (Calbiochem; catalog no. 344289 and 344291), Adenosine 5'-triphosphate disodium salt solution (Sigma; catalog no. A6559).

### Immunizing Peptide Library Synthesis

Three synthetic peptide libraries have been synthesized, consisting of either histidine or a non-hydrolyzable pHis analogue flanked by randomized amino acids (glycine [R=H] and alanine [R=CH3]), to promote generation of sequence-independent anti-pHis Abs. Each library is a complex mixture of peptides that are acylated at the N-terminus and contain L-cysteine for coupling to KLH. The three libraries each comprise polypeptides 10 amino acid in length and of the general structure: 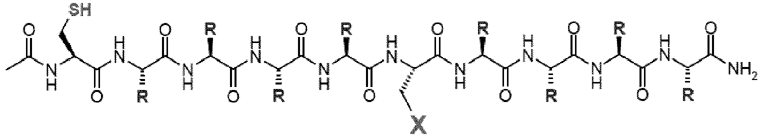 wherein
R is independently Gly or Ala (randomly assigned);
X is His, 1-pTza or 3-pTza (pTza = phosphoryltriazolylalanine);
each polypeptide is acylated at the N-terminus; and each polypeptide comprises an L-Cysteine (e.g., at or near the N- or C-terminus) for chemical ligation to a carrier protein (e.g., KLH). The total number of polypeptide combinations is 2⁸ X 3 = 768, and thus each of the three libraries (His, 1-pTza and 3-pTza contain 256 unique polypeptides

The library incorporating histidine will be used for negative selection of Abs that recognize non-phosphorylated histidine. The immunizing peptide libraries, one for each isomer, contain the non-hydrolyzable analogues, 1-pTza & 3-pTza, respectively. The pTza peptide libraries were coupled to KLH through their L-cysteine by the heterobifunctional linker m-maleimidobenzoyl-N-hydroxysuccinimide ester (MBS) according to previously published methods (Using Antibodies: A Laboratory Manual by Ed Harlow, Ch 5) wherein the carrier KLH is activated and purified prior to the coupling.

### Rabbit Immunization

In general, rabbits were immunized every three to four weeks and serum was collected ten days after each immunization. Each rabbit was injected intradermally with 0.5 ml antigen mixed with 0.5 ml adjuvant where Complete Freund's Adjuvant is used for the initial injection, and each subsequent injection is made with Freund's Incomplete Adjuvant.

### Peptide Dot Blots

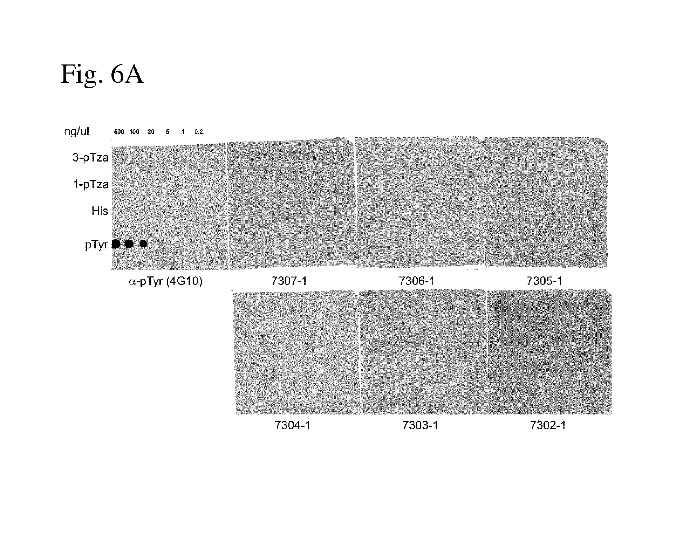

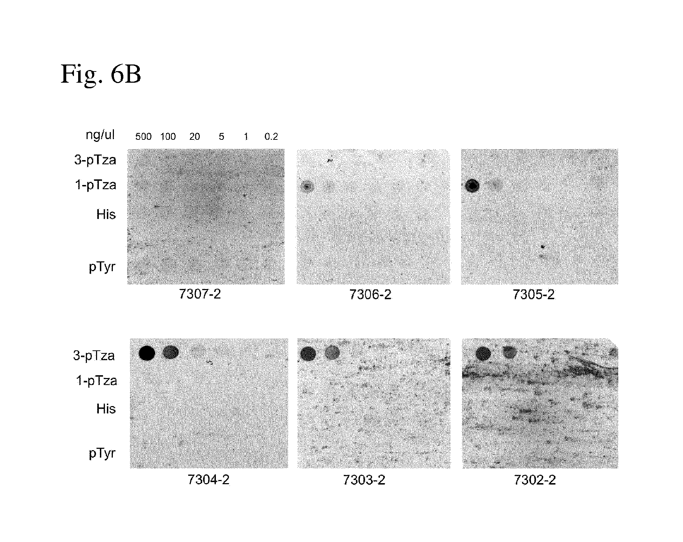

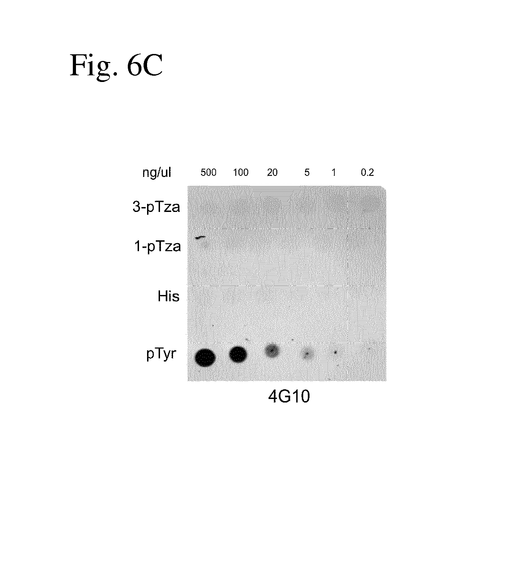

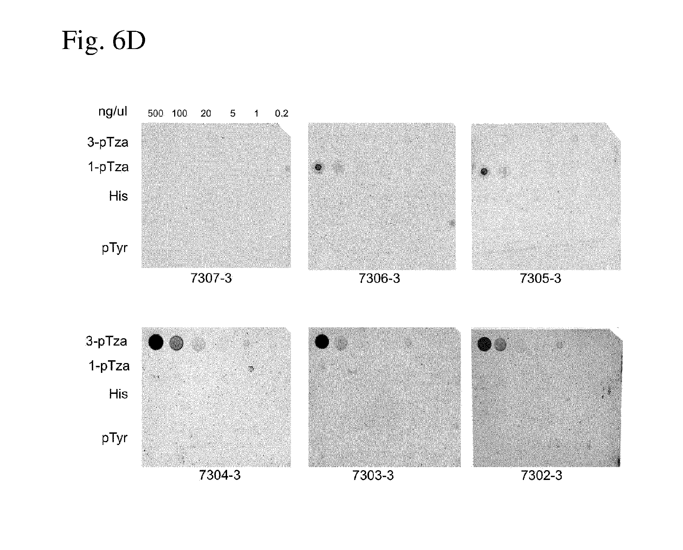

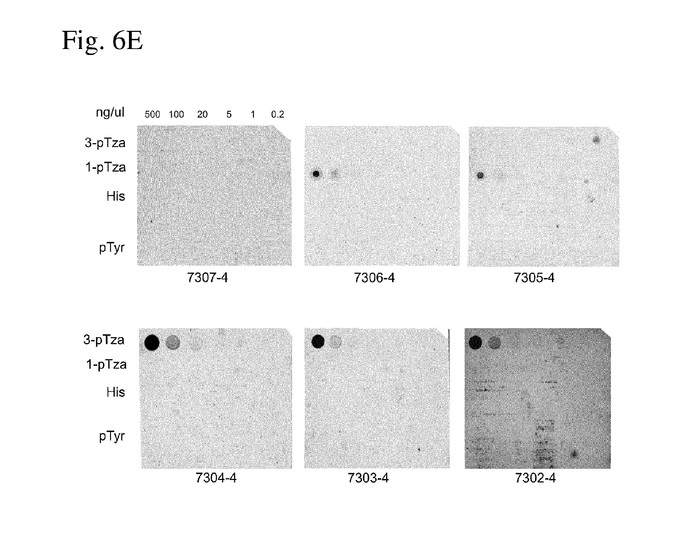

Each of the three peptide libraries (0.5 mg) was dissolved in PBS and used to make a 1ug/ul stock solution. Six fold, 1:5 serial dilutions were performed with a starting concentration of 500 ng/ul for each peptide library. 1 ul of each peptide dilution was spotted on nitrocellulose membranes (Whatman Protran BA85) and allowed to dry for 30 min. Membranes were then blocked for 1 hr at room temperature in blocking buffer (0.1 % Casein Block [BioRad] in 0.2X PBS) prior to overnight incubation at 4°C with rabbit antisera diluted 1:1000 in blocking buffer supplemented with 0.1 % Tween-20. Membranes were washed with 0.1 % TBST and incubated Alexa Fluor 680 goat anti-rabbit IgG secondary antibodies (Invitrogen, Cat. A21109) diluted at 1:20,000 in 0.1 % TBST for 45 min at room temperature. Membranes were then washed and imaged using the LI-COR Odyssey Infrared Imaging System. Figure 6 (A-E) shows the results of such dot blots for the first four bleeds of the six rabbits used. Rabbits are identified by numbers (7302-7307). Rabbits 7302-7304 were injected with the PTza-3 analog; rabbits 7305-7307 were injected with the PTza-1 analog.

### Recombinant Nm23 Expression and Purification

The pGEX-Nm23-H1 plasmid was created by inserting Nm23-H1 into the BamH1/EcoRI restriction sites of the GST fusion vector pGEX-6-P1 using the following primers; Forward 3'-GATCGGATCCATGGCCAACTGTGAGCGTAC-5' and Reverse 3' GATCGAATTCTCATTCATAGATCCAGTTCTC-5'. Rosetta 2 (DE3) competent cells were transformed with pGEX-Nm23-H1 and an overnight culture of LB Amp [50] / Chl [34] was inoculated with a single colony for protein expression. The next day, a 1:100 dilution of overnight culture was used to inoculate 200 mL LB Amp [50] / Chl [34] to an OD₆₀₀ of 0.2 and protein expression was induced at OD₆₀₀ = 0.6 with 1 mM IPTG for 3 hrs at 30°C. E.coli were pelleted at 5,000 x g for 10 min at 4°C and stored at -80°C until protein purification.

Purification of Nm23-H1: bacterial pellets were thawed on ice, resuspended in 1 mL GST Lysis Buffer (50 mM Tris-HCl pH 8.0, 100 mM NaCl, 1 mM DTT) / 50 mL culture and sonicated to lyse cells (8 sec. pulse, rest 1 min, 4x (on ice)). Sonicated lysates were spun at 14,000 x g for 30 min at 4°C to pellet insoluble material. The Glutathione Resin (1.5 mL slurry per 200 mL culture) was equilibrated by washing with GST Lysis Buffer. The washed resin was resuspended with 2 mL GST Lysis Buffer / 200 mL culture and the supernatant from bacterial lysates was transferred from ultracentrifuge tubes to fresh 15 mL conical tubes, combined with the washed resin and rotated 1.5 hrs at 4°C. The bound resin was pelleted (1000 x g for 2 min at 4°C) and washed 3 x with 10 mL GST Lysis buffer.

Ice-cold PreScission Protease Buffer (50 mM Tris-HCl pH 7.0, 150 mM NaCl, 1 mM EDTA, 1 mM DTT) was used to resuspend the washed, pelleted resin and 2.5 ul PreScission Protease (5U/200mL culture) was added and incubated overnight at 4°C to cleave GST and release purified Nm23-H1 from the resin. The resin was then pelleted (1000 x g for 5 min at 4°C) and the supernatant was carefully removed, transferred to a concentrator/desalting column (Millipore, Ultrafree 0.5 - 5K MWCO) in 500 ul increments and spun at 12,000 x g, 4°C until the volume reached 50 ul. Buffer exchange was performed by adding 3 x 450 ul Storage Buffer (50 mM Tris-HCl pH 8.0, 100 mM NaCl, 1 mM DTT) to the 50 ul concentrated sample. Purified, concentrated Nm23-H1 proteins were brought to 10% Glycerol in Buffer B, aliquoted, frozen in liquid nitrogen and stored at -80°C until use.

### Nm23 Autophosphorylation Assay

Autophosphorylation of recombinant Nm23-H1 was performed using ATP as the phosphate donor for Nm23-H1; as a negative control reactions were performed lacking ATP. 1 ul recombinant Nm23-H1 [1 ug/ul] was added per 50 ul TMD buffer (20 mM Tris-HCl pH 8.0, 5 mM MgCl2, 1 mM DTT). ATP (0.1 - 1 mM) was added fresh from aliquots of a 10 mM stock solution. Reactions were incubated at room temperature for 10 min and immediately stopped by addition of 5X pH 8.8 Sample Buffer (50 mM Tris-HCl pH 8.8, 2% SDS, 100 mM DTT, 10% Glycerol, 10 mM EDTA, 0.01% bromophenol blue).

Reactions were not heated prior to loading on 12.5% polyacrylamide gels (made with minimal pH 6.8 stacking gels) to preserve phosphorylation of histidine by limiting exposure to heat and low pH. The acid treated controls were performed by adding 15 ul 1M HCL to completed autophosphorylation reactions with incubation at 37°C for 15 min, followed by neutralization with 15 ul 1M NaOH. All gels were run at 80-100V for approximately 3 hrs at 4°C to prevent generation of heat. Proteins were transferred at 30V to PVDF membranes, overnight at 4°C for immunoblotting by standard procedures. The detection limit for anti-pHis antisera was assayed by making a 1:2 serial dilutions (200, 100, 50, 25, 12 and 6 ng) of autophosphorylated Nm23-H1 kinase in TMD buffer. 5X pH 8.8 Sample Buffer was added to each dilution followed by analysis by SDS-PAGE (without heating) and immunoblotting with the pHis antisera diluted at 1:1000 in blocking buffer supplemented with 0.1 % Tween-20. Membranes were washed and imaged as described above using the LI-COR Odyssey Infrared Imaging System.

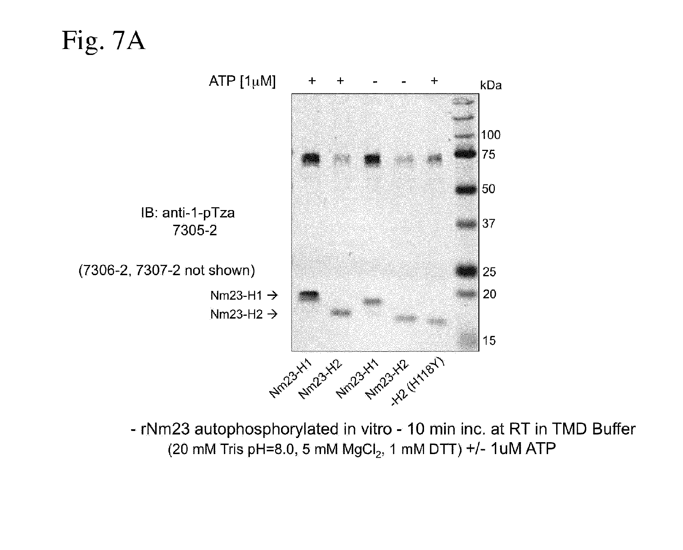

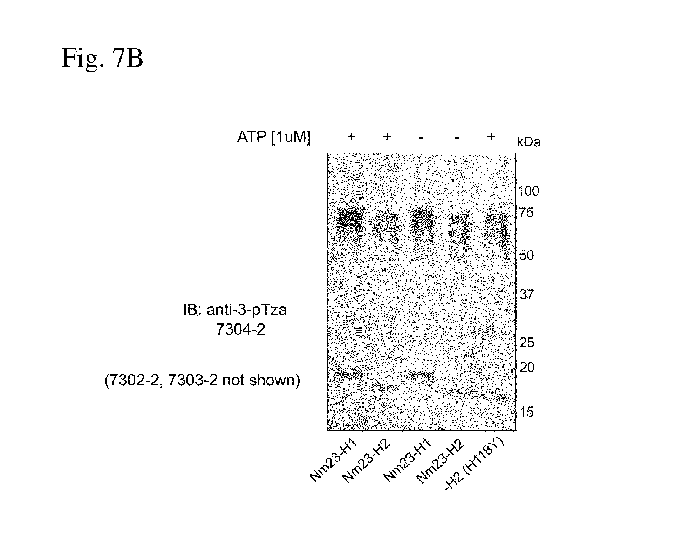

Figures 7-9 show preliminary tests (Western blots) of the antisera's abilities to recognize the autophosphorylated Nm23-H1.

### Results

The peptide libraries have been coupled to KLH via an N-terminal Cys present in the pHis analog containing peptides and an immunization protocol has been initiated for three rabbits per immunogen. Bleeds were screened for pHis detection by dot blot using the immunizing peptides. Unphosphorylated versions of the peptide libraries (with His in place of the pHis analog) were used as negative selection of Abs and served as a negative control for dot blot screening of antisera prior to purification. Anti-1- or -3-pHis-specific Abs were purified from antisera using first an affinity column consisting of immobilized unphosphorylated peptides followed by using affinity columns consisting of the immobilized libraries, which include one/either of the non-hydrolyzable analogues.

To remove Abs crossreactive with other phosphoamino acids, the column was washed with sodium phosphate followed by pSer, pThr and pTyr. That some anti-pTyr Abs cross-react with pHis demonstrates that pHis is indeed antigenic and using the approach outlined above Abs that specifically recognize pHis and not other phosphoamino acids were generated. See Figs 6A-6E demonstrating polyclonal antibodies specific for the 3-pHis library polypeptides (7306 and 7305) or 3-pHis library polypeptide (7302, 7303 and 7304) were generated.

To validate Ab specificity, autophosphorylated pH118 Nm23-H1/-H2 and dot blots with *in vitro* phosphorylated pHis substrates (e.g. histone H4) were used as positive controls. Recombinant GST-Nm23 proteins were expressed and purified using glutathione resin with subsequent cleavage of the GST using PreScission protease (See Fig. 4). *In vitro* autophosphorylation was conducted on the purified recombinant Nm23-H1 as described in the material and method section. Autophosphorylated rNm23 is detected by anti-1-pTza 7305 antisera in an immunoblot (see Fig. 7A); but anti-3-pTza 7305 antisera only produced background/non-specific binding to rNm23 (See Fig. 7B). Presumably this is due to a conserved Glu-NH hydrogen bond present at E129 in human Nm23 restricting autophosphorylation to only N1.

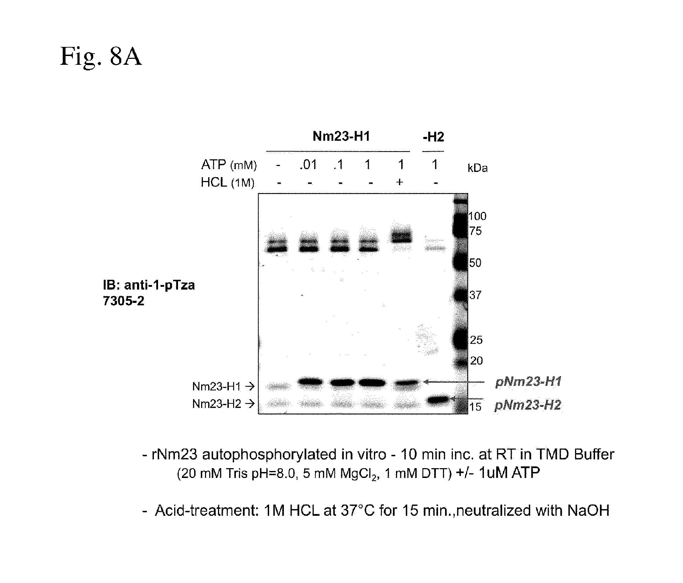

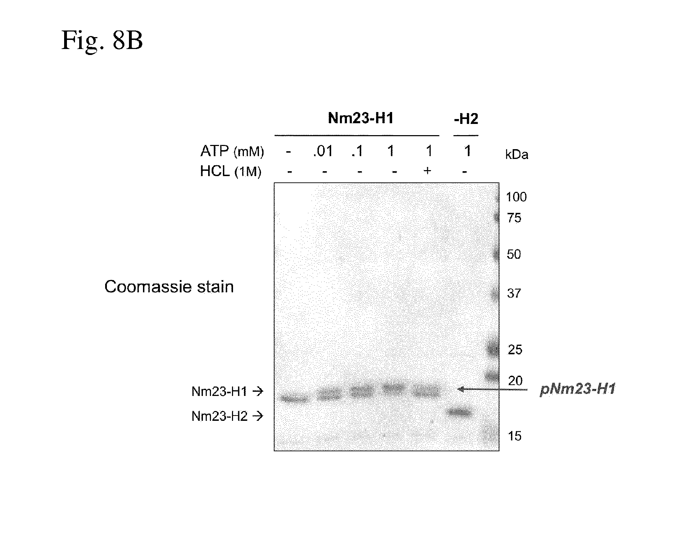

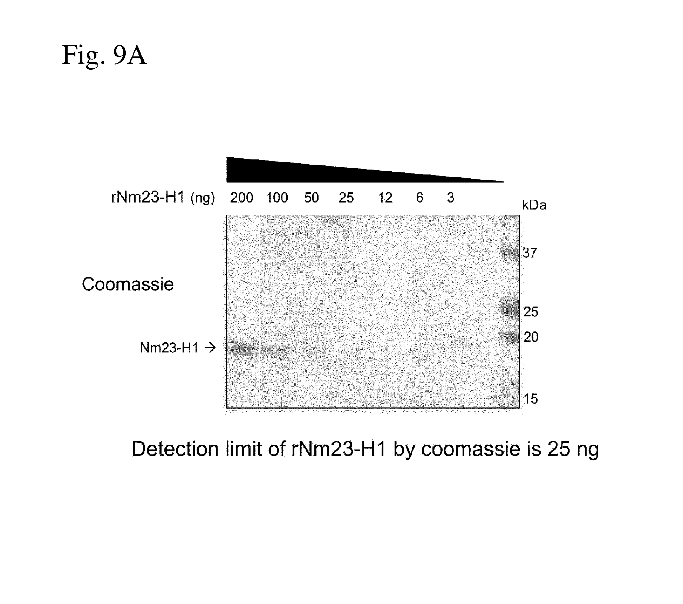

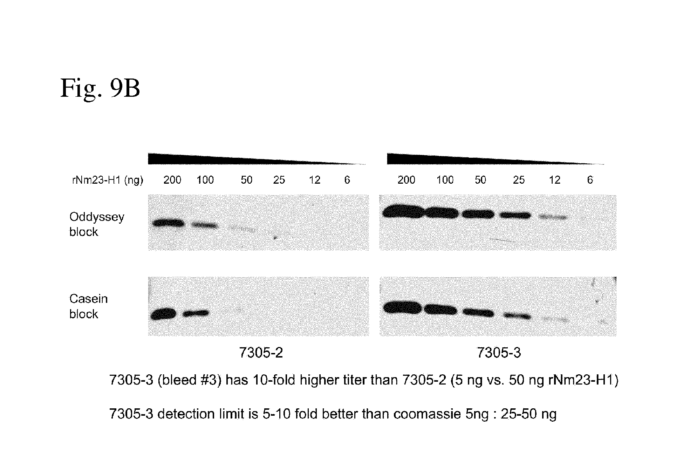

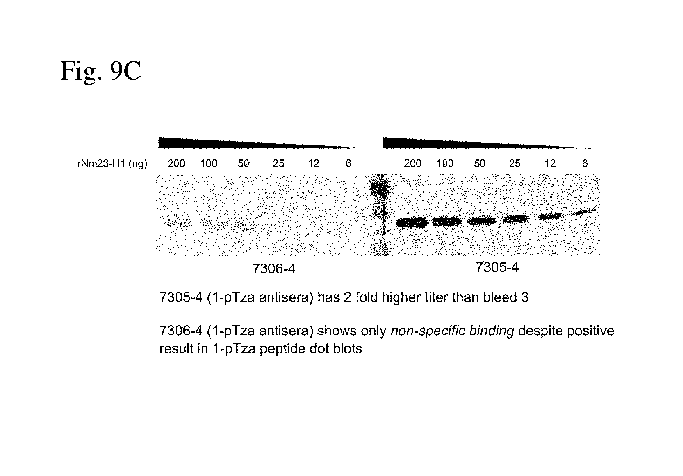

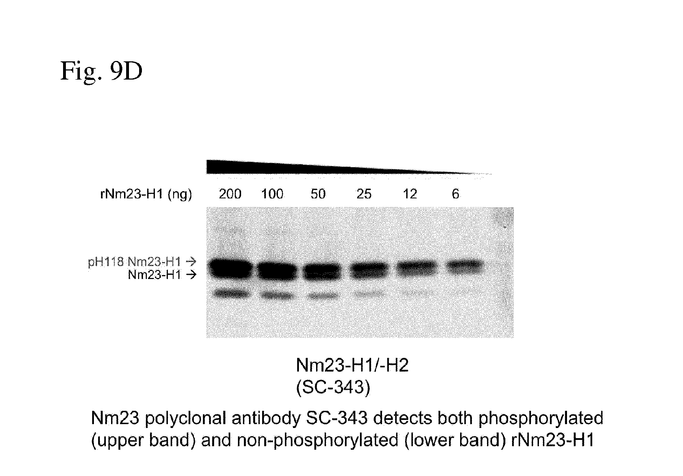

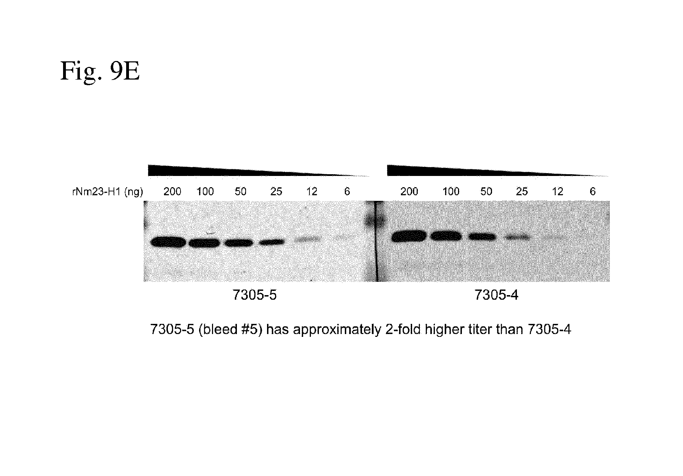

The detected rNm23 autophosphorylation is reversible. Acid treatment (1M HCl at 37°C for 15 min followed by neutalization with NaOH) of the autophosphorylated rNm23 significantly reduces the binding of anti-1-pTza 7305-2 to the pNm23 band (see Fig. 8A). Coomassie stained gel demonstrates the presence of the rNm23-H1/H2 and shows a reduction in the phosphorylated pNm23-H1 in the acid treated lane (See Fig. 8B). Figs. 9A and 9B show the coomassie detection limit vs the anti-1-pTza antisera detection limit, respectively for rNm23-H1 autophosphorylation. Fig. 9C shows the titre levels continue to increase in the fourth bleed of 7306 and 7305 rabbits, with Fig. 9D showing the 7306 and 7305 fourth bleed detection limits. Fig. 9E compares the titre of bleed 4 vs bleed 5 for detecting rNm23-H1 autophosphorylation using 7305 anti-sera.

### Stable Cell Lines Expressing FLAG-Nm23-H1

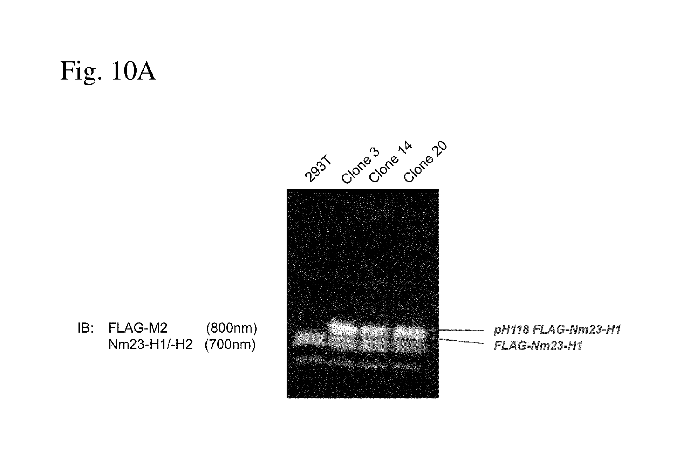

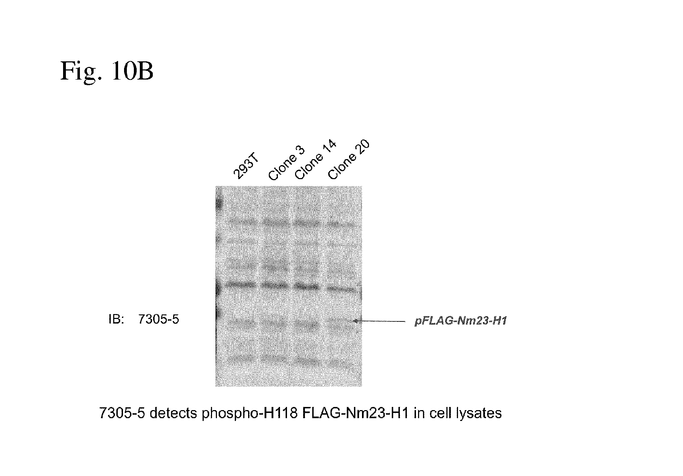

293T cell were transfected with DNA constructs expression FLAG-Nm23-H1 and stable lines were identified. The clones with the highest expression relative to tubulin was then used for all subsequent pHis immunoprecipitation experiments. Screening cell lines (See Fig. 10A and 10B) revealed that clone 20 exhibited the highest expression of Nm23-H1 relative to tubulin. As shown in Fig. 10B 7305-5 anti-sera can detect pFLAG-Nm23-H1.

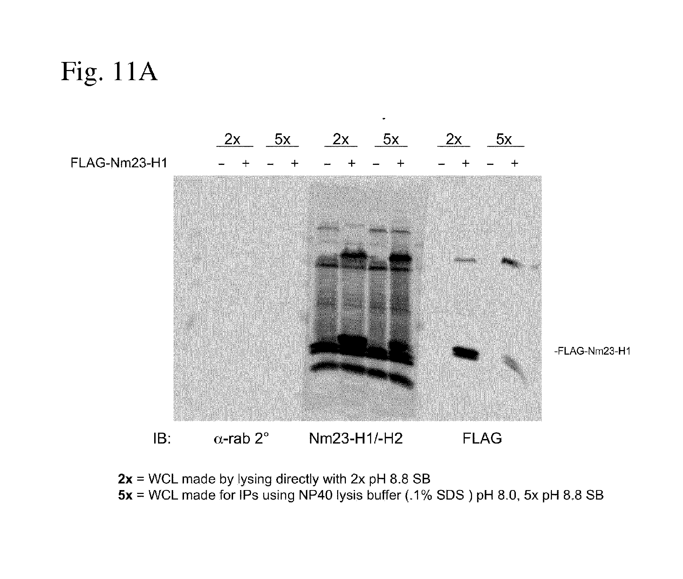

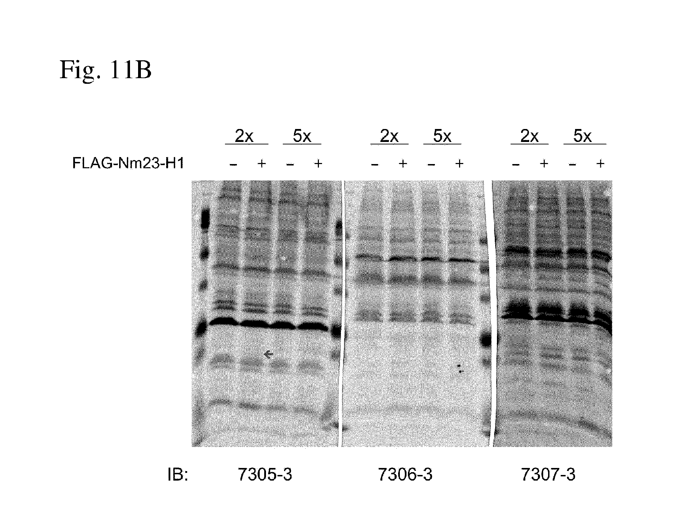

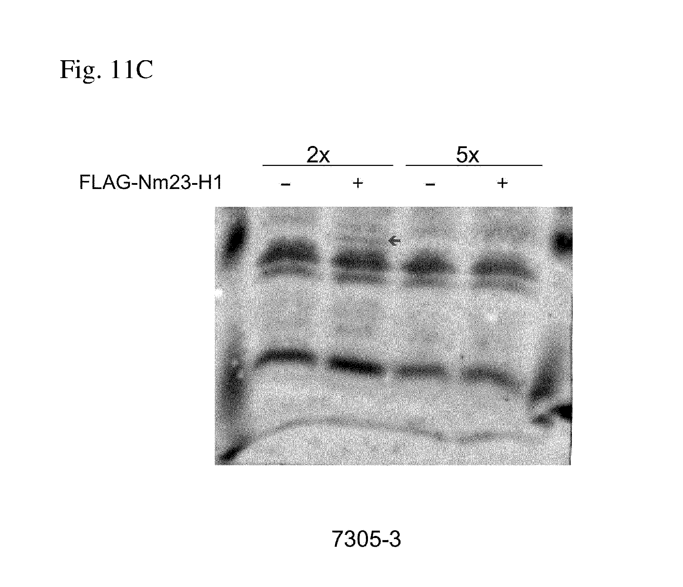

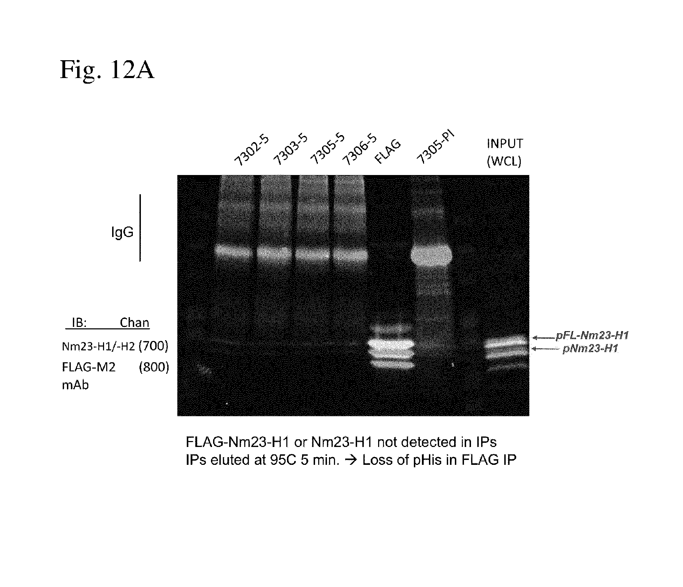

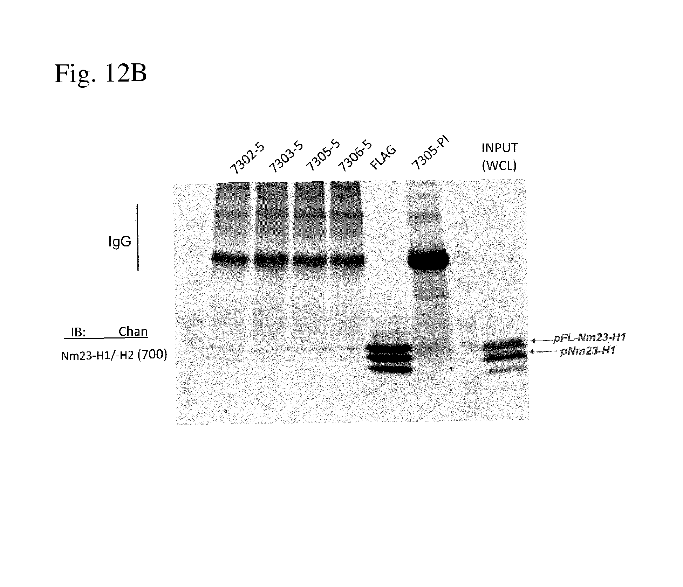

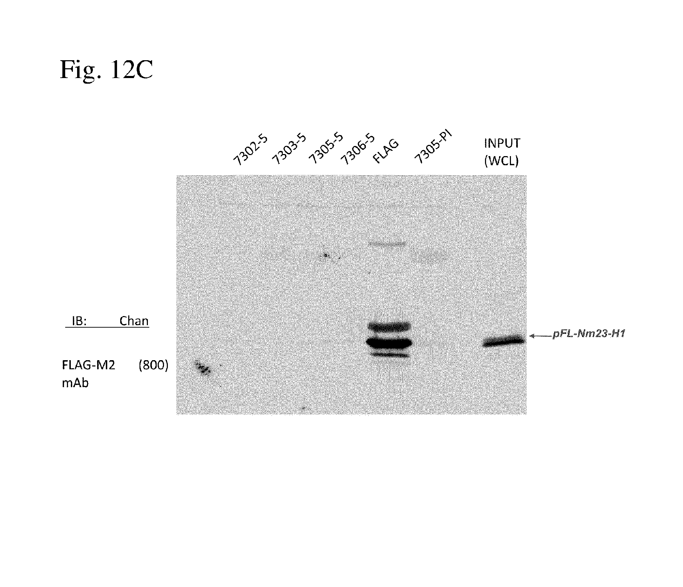

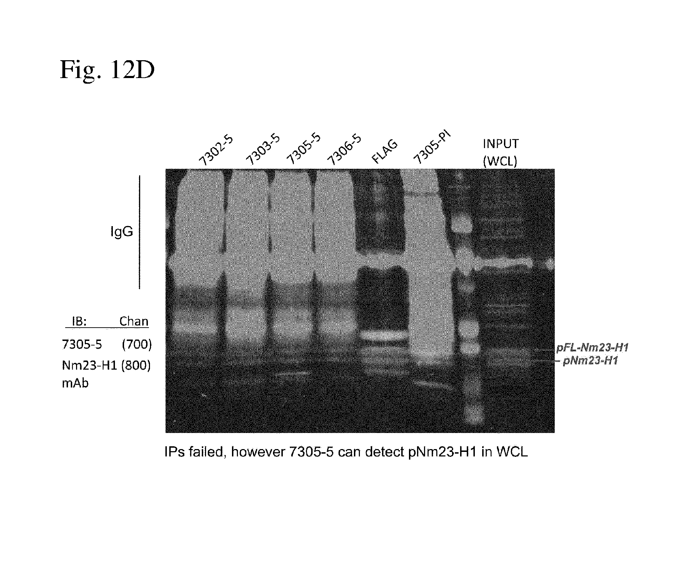

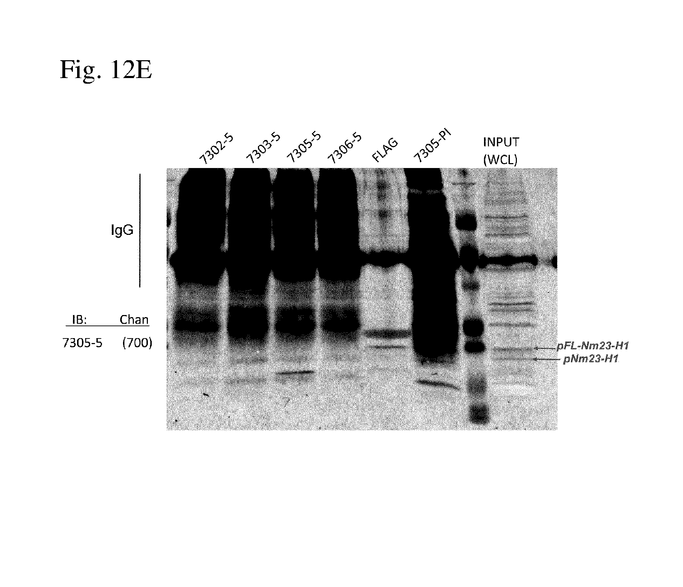

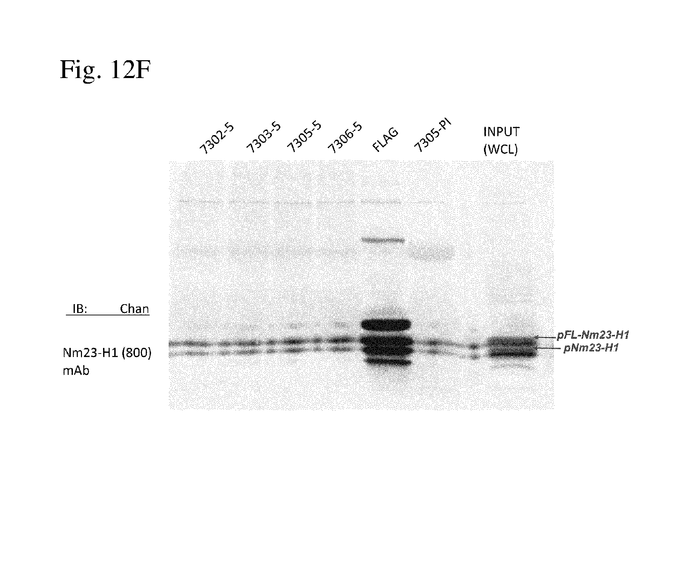

Next experiments were conducted to immunoprecipitate FLAG-Nm23-H1 from cell lysates. Results are shown in Figs. 11A-11C. A faint band can be detected using 7305-3 anti-sera. One possible reason for the discrepancy between the immunoblot data and the imunoprecipitation results may relate to the fact that pH118 resides inside the active site of the kinase. Therefore, Nm23-H1 may need to be denatured in order for the antibodies to access pH118. Stable FLAG-Nm23-H1 293Ts were used for IP with bleed 5 anti-pHis Abs from 7305-5. These results are presented in Figs 12A-12F.

In general, pHis substrates will be identified by immunoprecipitation (IP) and immunoblotting (IB) with anti-pHis Abs, or by pHis peptide enrichment and mass spectroscopy (MS) analysis. pHis substrates with relevance to cancer and metastasis will be identified by comparing cells with high and low Nm23 expression, followed by annotation using GO and pathway analysis to prioritize which substrates to study further.

### Endogenous pHis substrates from cancer cells.

PHis-containing proteins from whole cell lysates (buffered to pH 8 to stabilize pHis) made from cancer cell lines or primary tumor samples with high metastatic potential (i.e. low Nm23 expression; breast, ovarian, hepatocellular, cervical and gastric carcinomas or low metastatic potential (i.e. high Nm23 expression) will be immunoprecipitated using anti-pHis Abs. The immunoprecipitated proteins will be digested into peptides and enriched for pHis prior to MS analysis. Protocols for the selective extraction of peptides containing phosphomonoester residues (pSer, pThr and pTyr) using immobilized Fe(III) or Ga(III) ion affinity chromatography are well established. A similar protocol has been developed for enrichment and identification of pHis peptides based on use of appropriate pH conditions, and immobilized Cu(II) ion affinity chromatography (Cu(II)-IMAC) followed by matrix-assisted laser desorption/ionization time-of-flight mass spectrometry (MALDI-TOF MS). The synthetic pHis immunogen peptides and recombinant, autophosphorylated Nm23 proteins **(****Fig. 2A****)** will be used to optimize identification of pHis-containing peptides by MS. His phosphorylation levels due to Nm23 His kinase activity are expected to be reduced in highly metastatic cell lines relative to non-metastatic cell lines, reflecting the reduced Nm23-H1/-H2 expression in these cells. We will also deplete PHPT1 pHis phosphatase using shRNA in an attempt to increase the levels of pHis in proteins, analogous to using pervanadate to enhance pTyr levels.

### Overexpression of histidine kinases in cells.

Autophosphorylation of His118 (H118) on Nm23-H1 and Nm23-H2 is required for their kinase activity. Line IV human melanoma cells have high metastatic potential and low Nm23 expression. Line IV cell lines stably expressing Nm23-H1, Nm23-H2 and their respective, catalytically inactive mutants (H118Y) have been obtained. Cell lysates or tryptic digests will be probed for pHis with Abs and analyzed by MS as described in above. In vitro kinase assays using purified proteins will be used to validate selected substrates of interest identified by MS. Mutagenesis of specific His residues to generate pHis-deficient mutants will be used to investigate the *in vivo* function of pHis for each respective target by expression in a null background (e.g. knockout MEFs) or by re-expression in depleted cells (e.g. si/shRNA-treated cells) to determine if the pHis-deficient mutants rescue WT protein function.

Nm23-H1 and Nm23-H2 genes are 88% identical and yet have distinct substrates and functions. Nm23 genes are highly conserved between human and rodents and have similar organization and tissue expression. Nm23-H1-/- and Nm23-H2-/- mice are viable; however, H1/H2-deficient mice are not, suggesting some functional overlap and that compensation occurs. Tissues from Nm23-H1-/- and H2-/- mice will be analyzed for pHis (using methods outlined above, as well as straight IB) and compared with tissues of WT mice to determine substrates specific for Nm23-H1, Nm23-H2 or both. Selected targets identified in this way will be further investigated through the use of si/shRNA knockdown and re-expression of WT or pHis-deficient mutants to examine effects of His phosphorylation on tumor metastasis (e.g. migration, invasion, anchorage-independent colony formation, and tumorigenesis in nude mice).

## Claims

1. A peptide comprising the structure
Z-W-Y
wherein
Z is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈;
W is an amino acid selected from 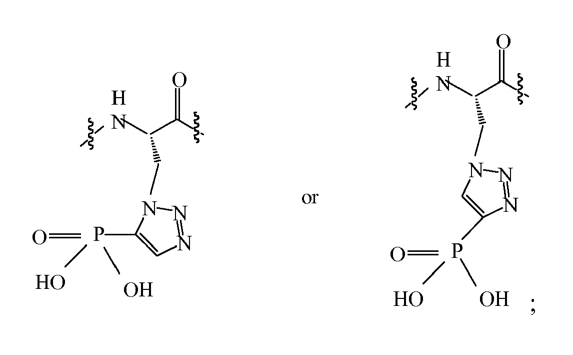 Y is a sequence selected from the group consisting of X₁, X₁X₂, X₁X₂X₃, X₁X₂X₃X₄, X₁X₂X₃X₄X₅, X₁X₂X₃X₄X₅X₆, X₁X₂X₃X₄X₅X₆X₇ and X₁X₂X₃X₄X₅X₆X₇X₈; and
X₁, X₂, X₃, X₄, X₅, X₆, X₇, and X₈ are independently alanine or glycine.

2. The peptide of claim 1 further comprising a single cysteine, tyrosine or lysine.

3. The peptide of claim 1 further comprising an N-terminal cysteine.

4. The peptide of claim 3 wherein the N-terminal amine is acylated and the C-terminal carboxylic acid group is replaced with an amide.

5. The peptide of claim 4 wherein both Z and Y are independently X₁X₂X₃X₄.

6. The peptide of claims 2-5 covalently coupled to a carrier protein.

7. The peptide of claim 6 wherein the carrier protein is keyhole limpet hemocyanin linked through the side chain of the cysteine, tyrosine or lysine amino acid of said peptides.

8. The peptide of claim 6 emulsified in an adjuvant.

9. The peptide of claim 8 wherein the adjuvant is Freund's Complete or Freund's Incomplete adjuvant.

10. A composition/library comprising a plurality of different peptides of claim 2.

11. The composition of claim 10 comprising 256 different peptides
wherein each of said 256 peptides comprises the structure Cys-Z-W-Y.

12. The composition of claim 10 wherein said peptides are covalently coupled to a carrier protein through the side chain of the cysteine amino acid of said peptides, and emulsified in an adjuvant.

13. A method for producing antibodies, said method comprising
i) immunizing a host with a composition of claims 8 or 12; and
ii) obtaining an antibody-containing host serum produced as a response to said immunization.

14. The method of claim 13, wherein the host is immunized with the composition of claim 6.

15. The method of claim 14 wherein the composition further comprises a carrier protein and an adjuvant.

16. The method of claim 15 wherein the composition comprises 256 different peptides wherein each of said 256 peptide comprises the structure Cys-Z-W-Y.

17. An antibody produced by the method of claim 16, wherein the antibody specifically binds to any peptide comprising the structure of claim 1.

18. An antibody that specifically binds to a peptide of claim 1, or a peptide derivative of claim 1 wherein W is phosphohistidine, but does not specifically bind to a peptide comprising a peptide derivative of claim 1 wherein W is histidine.

19. The antibody of claim 17, wherein said antibody is polyclonal.

20. The antibody of claim 17, wherein said antibody is monoclonal.

21. A hybridoma cell line producing the antibody of claim 17.

22. A method for detecting proteins comprising a phosphohistidine in a biological sample, said method comprising the steps of:
(a) incubating/mixing a biological sample with at least one antibody of claim 14 under conditions suitable for formation of a reagent-antibody complex; and
(b) detecting the presence of said complex in said sample, wherein the presence of said complex indicates the presence of a protein comprising a phosphohistidine in said sample.

23. The method of claim 22 wherein the antibody is labeled.

24. A kit for the detection of proteins comprising a phosphohistidine, said kit comprising
an antibody of claim 18; and
a secondary antibody conjugated to a detectable label.

25. An antibody that specifically binds to peptides comprising a phosphohistidine, but fails to specifically bind to an identical peptide that comprises a histidine in place of the phosphohistidine.

26. The antibody of claims 25 wherein antibody is a monoclonal antibody.
